# FASCICULE DE BREVET EUROPEEN

(11) **EP 0 673 357 B1**
(45) Date de publication et mention de la délivrance du brevet: **09.04.1997**
(21) Numéro de dépôt: 94902022.6
(22) Date de dépôt: 10.12.1993
(51) Int. Cl.: C07C 55/14, C07C 55/12, C07C 55/10, C07C 51/06

(54) **PROCEDE DE TRAITEMENT D'UN MATERIAU COMPRENANT UN POLYMERE PAR HYDROLYSE**
VERFAHREN ZUR HYDROLYSEBEARBEITUNG VON EINEM POLYMERISAT ENTHALTENDEM MATERIAL
PROCESS FOR THE HYDROLYSIS TREATMENT OF A MATERIAL COMPRISING A POLYMER

(30) Priorité: 11.12.1992 FR 9215206
(43) Date de publication de la demande: 27.09.1995
(73) Titulaire: RHONE-POULENC CHIMIE, 92408 Courbevoie Cédex (FR)
(72) Inventeur: DOS SANTOS, Emmanuel, F-69320 Feyzin (FR); METIVIER, Pascal, F-69110 Sainte-Foy-les-Lyon (FR); GUBELMANN, Michel, F-75016 Paris (FR)
(74) Mandataire: Esson, Jean-Pierre
(86) Numéro de dépôt international: FR9301232
(87) Numéro de publication internationale: WO9413616

(56) Documents cités:
- DE-C- 867 546
- US-A- 2 407 896

## Description

La présente invention concerne un procédé de traitement d'un matériau comprenant un polymère pour récupérer au moins partiellement les monomères de formation du polymère.

Elle se rapporte plus particulièrement à un procédé de traitement d'un matériau comprenant un polymère contenant des fonctions amides par hydrolyse oxydante de ces fonctions amides pour régénérer des monomères utilisables pour la synthèse de polymères identiques ou différents.

Les matières synthétiques et parmi ceux-ci les polymères contenant des fonctions amides tels que les polyamides et plus particulièrement le polyamide 6.6 ou le polyamide 6, sont de plus en plus utilisés pour la réalisation d'articles variés tels que fibres textiles, fils industriels techniques, filaments ou articles moulés comme pièces pour l'électricité, l'électronique ou l'automobile. Ces matériaux synthétiques sont généralement utilisés sous forme de compositions comprenant en plus du matériau synthétique, différents additifs pour, par exemple, augmenter leur stabilité thermique, ou aux rayonnements, améliorer leurs propriétés mécaniques, électriques ou électrostatiques, des colorants, des pigments. Ces additifs sont de nature très variée et peuvent être aussi bien des composés organiques que minéraux.

En outre, notamment pour la réalisation d'articles moulés, les compositions comprennent des charges souvent minérales telles que les fibres de verre, du talc, de l'argile etc...

Les matériaux ainsi produits peuvent être détruits après usage. Un des moyens de destruction classique est l'incinération qui permet de récupérer de l'énergie. Toutefois, il est également proposé de recycler ces matériaux. Un des moyens de recyclage consiste à dépolymériser le polymère ou matière synthétique en des composés de degré de condensation plus faible et si possible régénérer les monomères de départ pour pouvoir les recycler dans les procédés de polymérisation. Un des procédés proposés pour le recyclage du polyamide est un procédé consistant à hydrolyser le polymère en milieu basique. Toutefois, ce procédé ne permet pas de régénérer les monomères et requiert généralement une séparation préalable entre le polymère et les charges. En outre, les additifs présents dans le polymère, notamment quand ils sont de nature organique peuvent polluer les produits récupérés après l'hydrolyse, cette pollution pouvant empêcher leur recyclage.

Un des buts de la présente invention est notamment de proposer un procédé permettant de traiter des matériaux pouvant comprendre des charges et/ou des additifs, pour hydrolyser les fonctions amides du polymère contenu dans le matériau afin de régénérer d'une part l'acide dicarboxylique de départ et d'autre part de produire un ou plusieurs acides carboxyliques dont la structure chimique correspond sensiblement à celle des monomères diamines ou aminoacides de départ.

A cet effet, l'invention propose un procédé de traitement d'un matériau comprenant un polymère contenant au moins des fonctions amides. Ce procédé se caractérise en ce qu'il consiste à soumettre le matériau à une hydrolyse en présence de groupements nitreux dissouts dans le milieu d'hydrolyse et à une oxydation .

Selon un mode de réalisation préférentiel, le milieu d'hydrolyse est un milieu oxydant.

Selon une caractéristique de l'invention, les groupements nitreux sont obtenus par décomposition chimique ou thermique d'au moins un composé comprenant ces groupements.

Ainsi, on peut citer, à titre d'exemple, comme composés générateurs de groupements nitreux, l'acide nitrique, les nitrites alcalins, métalliques ,les vapeurs nitreuses (NOₓ)ou analogues.

Le milieu d'hydrolyse est un milieu aqueux, présentant un pH acide de préférence inférieur à 1.

Dans un mode de réalisation préféré, la concentratrion en H⁺ dans le milieu est au moins égale à 1 mole/l (1N).

Le procédé de l'invention est avantageusement réalisé par maintien du milieu d'hydrolyse à une température inférieure à 100°C, de préférence comprise entre 40 et 100°C. Toutefois, le procédé de l'invention peut être mis en oeuvre sous pression à une température supérieure à 100°C, par exemple à une température comprise entre 100 et 200°C .

La durée de réaction est déterminée pour avoir une hydrolyse complète du polymère et est, par exemple, de quelques heures (1 à 10 heures).

Il peut être également avantageux d'avoir au-dessus du milieu d'hydrolyse, une atmosphère chargée en vapeurs nitreuses.

Après réaction, les résidus solides sont avantageusement éliminés, notamment par filtration.

Le mélange peut être ensuite refroidi et concentré pour permettre la cristallisation des acides dicarboxyliques formés. Cette méthode de récupération des acides carboxyliques est la plus couramment utilisée, mais la séparation de ces acides peut être obtenue par tout procédé approprié.

Selon un autre mode de réalisation, le traitement d'hydrolyse de l'invention est réalisé en présence de catalyseurs d'oxydation . Des catalyseurs convenables sont, par exemple, des composés métalliques tels que des composés de cuivre, vanadium, titane, fer, cobalt, molybdène, nickel, ruthénium, manganèse, irridium ou analogue .On peut citer, à titre d'exemple, le vanadate d'ammonium, le nitrate de cuivre .

Les matériaux qui peuvent être traités par le procédé de l'invention sont tous les matériaux comprenant une matière synthétique polymérique, dont au moins une unité récurrente comprend au moins une fonction amide. Ainsi, le procédé d'hydrolyse de l'invention permet de couper les chaînes notamment au niveau de chaque fonction amide pour redonner au moins la fonction acide du monomère ou oligomère de départ et transformer au moins partiellement les monomères ou oligomères diamines de départ en composés à fonction dicarboxylique.

Le procédé de l'invention s'applique plus particulièrement, aux traitement des polymères issus d'au moins un monomère comprenant des fonctions acides et/ou amines non reliées directement à un noyau aromatique.

Les autres composés organiques contenus dans le polymère ou les composés non transformés en diacides carboxyliques subissent, dans le milieu d'hydrolyse de l'invention, une oxydation et sont donc généralement dégradés en carbone, CO₂, H₂O, CO.

Les matières synthétiques polymériques pouvant être traitées par le procédé de l'invention, sont par exemples les polyamides, polyétherestéramide, polyesteramide, polyétheramide, polyaramides .

De préférence, ce procédé s'applique au traitement des polyamides tels que les polyamides obtenus par polycondensation d'un diacide carboxylique linéaire avec une diamine linéaire comme le PA 6.6., PA.6.10, PA 6.12, ou entre un diacide carboxylique aromatique et une diamine linéaire ou aromatique comme les polytéréphtalamides, polyisophtalamides, polyaramides, les polyamides obtenus par polycondensation d'un aminoacide sur lui-même, l'amino-acide pouvant être généré par l'ouverture d'un cycle lactame tels que, par exemple PA 6, PA 7, PA 11, PA 12. Le procédé de l'invention est également convenable pour traiter les copolyamides dérivés notamment des polyamides ci-dessus, ou les mélanges de polyamides ou copolyamides.

Les matériaux pouvant être traités par le procédé de l'invention peuvent également comprendre d'autres composants que la matière synthétique polymérique. En effet, et cela représente un avantage important du procédé, il n'est pas nécessaire de séparer la matière synthétique polymérique des autres composants tels que charges, adjuvants, additifs, qui sont généralement mélangés à ladite matière pour améliorer certaines de ces propriétés, ou lui donner un aspect différent comme pour les colorants.

Ainsi, les charges généralement minérales qui sont mélangées à la matière synthétique pour notamment renforcer ses propriétés mécaniques, ne sont pas hydrolysées et précipitent dans le milieu d'hydrolyse. Il en est de même pour les additifs minéraux tels que l'oxyde de titane, usuellement employé comme matifiant dans les fils et fibres textiles ou techniques.

Les autres additifs organiques sont généralement hydrolysés, oxydés et transformés en carbone, oxyde de carbone, eau ou autres oxydes. Toutefois, il est également possible que la dégradation de certains de ces composés ne soit pas totale, mais les produits de dégradation résultants seront séparés des acides dicarboxyliques par cristallisation de ces derniers.

Ainsi, le procédé de l'invention permet de traitrer des matériaux bruts de leur récupération, tels que par exemples les fils pour tapis, bas en nylon, les pièces moulées. Les matériaux peuvent être traités tels quels ou après un traitement tel que séparation d'autres matériaux comme des inserts métalliques, ou réduction en petites particules par par exemple, broyage, découpage pour permettre une hydrolyse accélérée.

Le procédé de l'invention permet donc un recyclage des polymères tels que les polyamides sous forme de monomères diacides. Dans le cas du traitement du PA6.6, le procédé permet de récupérer l'acide adipique engagé au départ et de transformer au moins partiellement l'hexaméthylène diamine en acide adipique ou acides dicarboxyliques comprenant un nombre de carbone plus petit tel que l'acide glutarique, l'acide succinique, par exemple.

D'autres buts, avantages et caractéristiques de l'invention apparaitront plus clairement au vu des exemples donnés ci-dessous uniquement à titre indicatif.

Le procédé de l'invention, dans un mode de réalisation préféré, est réalisé dans un réacteur contenant un milieu d'hydrolyse constitué généralement par une solution aqueuse d'acide nitrique dans laquelle sont ajoutés du nitrite de sodium comme composé générateur de groupements nitreux et éventuellement des catalyseurs.

Le matériau à traiter est ajouté dans le milieu d'hydrolyse tel quel ou sous forme découpée ou broyée. Dans la plupart des cas, le matériau tel quel est engagé .

Le milieu réactionnel est maintenu sous agitation et à une température déterminée pendant environ 4 heures.

Après réaction, le milieu réactionnel est refroidi et dilué avec de l'eau. La quantité d'acide dicarboxylique, notamment d'acide adipique, glutarique et succinique quand le polymère de départ est du PA 6.6 ou PA 6, est déterminée par dosage par chromatographie en phase liquide.

Le rendement de transformation en diacides carboxyliques est exprimé par le rapport de la quantité d'acides dosée par rapport à la quantité théorique d'acides obtenue en prenant comme hypothèse que l'hydrolyse de la fonction amide régénère les fonctions acides de départ et transforme les fonctions amines en fonctions acides.

Le milieu réactionnel est éventuellement filtré pour éliminer les résidus solides, puis les acides dicarboxyliques sont séparés et récupérés par concentration et cristallisation, selon les techniques connues de cristallisation de l'acide adipique .

### EXEMPLE 1

Matériau traité : polyamide 6.6 pur contenant :
- 730 méq./kg NH₂ et
- 750 méq/kg COOH

Ce polymère a un degré de polymérisation DPn voisin de 12.

Le milieu d'hydrolyse comprend 5 ml de solution d'acide nitrique à 56 % en poids avec 76,8 mg de CuO et 4,5 mg de VO₃NH₄.

On ajoute dans le mélange 17,1 mg de NaNO₂.

Il se produit un dégagement important de vapeurs nitreuses dans le réacteur. 0,3265 g de PA 6.6 sont alors ajoutés et le milieu est maintenu à 70°C pendant 4 heures. (Cette masse de PA 6.6 contient potentiellement 1832 méq. de fonction COOH par Kg de PA et 1832 méq. de fonction NH₂ par Kg de PA).

Après refroidissement et dilution avec de l'eau, les acides dicarboxyliques produits sont dosés par chromatographie en phase liquide :
- acide adipique : 1,081 mol./Kg
- acide glutarique : 0,021 mol./Kg
- acide succinique : 0,101 mol./Kg
quantité théorique de diacides régénérés : 1,832 mol/Kg
rendements de transformation :
- acide adipique : 59,0 %
- acide glutarique : 1,1 %
- acide succinique : 5,5 %
- diacides totaux : 65,6 %

### EXEMPLES 2 A 6 ET EXEMPLES COMPARATIFS A ET B

Dans le tableau I ci-dessous sont rassemblés les résultats obtenus sur le traitement de PA 6.6 pur, avec des conditions opératoires différentes.

**Tableau I**

| essai | 1 | 2 | 3 | 4 | 5 | 6 | A | B |
|---|---|---|---|---|---|---|---|---|
| PA 6.6 | | | | | | | | |
| nb.. mmol AA (1) | 1,39 | 1,430 | 1,43 | 1,42 | 1,41 | 1,40 | 1;40 | 1,41 |
| nb. mmol HMDA (1) | 1,39 | 1,43 | 1,43 | 1,42 | 1,41 | 1,40 | 1,40 | 1,41 |
| AA libre résiduel (mmol) | 0,333 | 0,341 | 0,341 | 0,340 | 0,337 | 0,334 | 0,334 | 0,337 |
| NaNO₂ | 0,313 | 0,338 | 1,414 | 0,304 | 0,32 | 5,38 | --- | --- |
| Acide | HNO₃ | HNO₃ | HNO₃ | HNO₃ | HNO₃ | HNO₃ | HNO₃ (blanchi) | H₂SO₄ |
| | 56 % | 56 % | 56 % | 29 % | 56 % | 56 % | 56 % | 36,3 % |
| Catalyseur | Cu(NO₃)₂ | VO₃NH₄ | --- | Cu(NO₃)₂ | Cu(NO₃)₂ | --- | --- | Cu(NO₃)₂ |
| | VO₃NH₄ | --- | --- | VO₃NH₄ | | --- | --- | VO₃NH₄ |
| Température | 70°C | 70°C | 70°C | 70°C | 70°C | 70°C | 70°C | 70°C |
| Durée | 4h | 4h | 4h | 4h | 4h | 4h | 4h | 4h |
| mmol. diacide théorique | 2,120 | 2,17 | 2,17 | 2,16 | 2,15 | 2,13 | 2,13 | 2,15 |
| mmol AS | 0,140 | 0,155 | 0,109 | 0 | 0,102 | 0,179 | 0 | 0 |
| R % | 6,62 | 7,12 | 5,03 | | 4,75 | 8,42 | | |
| mmol AG | 0,017 | 0,022 | 0 | 0 | 0 | 0,038 | 0 | 0 |
| R % | 0,80 | 1,01 | | | | 0,81 | | |
| mmol AA | 1,082 | 1,110 | 1,099 | 0,496 | 1,06 | 1,136 | 0,008 | 0,024 |
| R % | 51,19 | 50,96 | 50,69 | 23 | 49,54 | 53,42 | 0,38 | 1,11 |
| mmol diacides | 1,239 | 1,287 | 1,208 | 0,496 | 1,16 | 1,353 | 0,008 | 0,024 |
| R % | 58,62 | 59,09 | 53,72 | 23 | 54,29 | 63,63 | 0,38 | 1,11 |
| AA = acide adipique | | | | | | | | |
| AG = acide glutanique | | | | | | | | |
| AS = acide succinique | | | | | | | | |
| HMDA = hexaméthylène diamine | | | | | | | | |

| | | | | | | | | |
|---|---|---|---|---|---|---|---|---|
| (1) nb de mmol de monomères engagé pour réaliser le polymère | | | | | | | | |

Ces exemples montrent clairement que la présence de groupements nitreux est nécessaire pour obtenir une hydrolyse de la fonction amide, (l'exemple A utilise un acide nitrique blanchi avec de l'acide sulfonique pour éliminer les groupements nitreux).

Par ailleurs, ils indiquent clairement que le procédé de l'invention permet d'une part, de régénérer une grande partie du monomère acide de départ, mais également de transformer une partie importante du monomère amine en composé diacide.

### EXEMPLES 7 ET 8

Ces exemples concernent le traitement de polymères différents :
- exemple 7 : polyamide 6.6 pur (en absence de monomères libres)
- exemple 8 : polyamide 6 pur.

Les conditions opératoires et les résultats sont rassemblés dans le tableau II.

**Tableau II**

| essai | 7 | 8 |
|---|---|---|
| Polymère | PA 6.6 Pur | PA 6 pur |
| nb.. mmol AA (1) | 1,341 | --- |
| nb. mmol HMDA (1) | 1,341 | --- |
| nb. mmol caprolactame (1) | --- | 3,15 |
| NaNO₂ | 0,338 | 0,338 |
| Acide | HNO₃ | HNO₃ |
| | 56 % | 56 % |
| Catalyseur | Cu(NO₃)₂ | Cu(NO₃)₂ |
| | VO₃NH₄ | VO₃NH₄ |
| Température | 70°C | 70°C |
| Durée | 4 h 40 | 4 h 35 |
| mmol. diacide théorique | 2,68 | 3,15 |
| mmol AS | 0,388 | 0,968 |
| R % | 12,48 | 30,68 |
| mmol AG | 0,058 | 1,452 |
| R % | 1,87 | 46,02 |
| mmol AA | 1,517 | 0,189 |
| R% | 48,78 | 5,99 |
| mmol diacides | 1,963 | 2,609 |
| R % | 63,12 | 82,70 |

| | | |
|---|---|---|
| (1) nb de mmol de monomères engagé pour réaliser le polymère | | |

### EXEMPLE 9

Le procédé décrit à l'exemple 1 est reproduit mais en remplaçant le polyamide 6.6 par un tissu de bas en "Nylon" PA 6.6.

Après traitement, le rendement de transformation du polyamide en diacide est exprimé en poids de diacides récupérés par kg de bas :

| | |
|---|---|
| acide adipique | 705 g/kg |
| acide glutarique | 2,4 g/kg |
| acide succinique | 129 g/kg |
| total diacides | 758 g/kg |
| Rendement de transformation | 75,8 %. |

### EXEMPLE 10

Le matériau traité est une pièce moulée réalisée à partir d'une composition en PA 6.6 et PA 6 chargée avec une charge minérale type talc et comprenant des additifs de stabilisation thermique conventionnel tels que Cul/KI.

Cette pièce a été introduite dans un milieu d'hydrolyse constitué par une solution d'acide nitrique à 56 % en poids dans laquelle ont été ajoutés du nitrate de cuivre, vanadate d'ammonium et du nitrite de sodium.

Le milieu a été maintenu à 70°C pendant 4 heures.

Après refroidissement, le milieu a été dilué par ajout d'eau.

Le milieu contient des composés insolubles de couleur blanchâtre qui sont éliminés par filtration.

La quantité de diacides dans le milieu d'hydrolyse récupérés après filtration est déterminée par chromatographie en phase liquide.

Les résultats obtenus sont :

| | |
|---|---|
| acide adipique | 1,562 mmol |
| acide glutarique | 0,336 mmol |
| acide succinique | 0,562 mmol |
| diacides totaux | 2,460 mmol |

Les rendements de transformation de polyamide engagés en diacides sont :
- polyamide engagé :

| | |
|---|---|
| PA 6.6 | 4,13 mol/kg de composition |
| PA 6. | 1,33 mol/kg de composition |

- diacides formés :

| | |
|---|---|
| acide adipique | 2,20 mol/kg de composition |
| acide glutarique | 0,47 mol/kg de composition |
| acide succinique | 0,79 mol/kg de composition |
| acides totaux | 3,46mol/kg de composition |

Le rendement de transformation des polyamides en diacides est de 63,4 %. Ces diacides sont ensuite récupérés par cristallisation.

Ainsi, par refroidissement du milieu d'hydrolyse filtré on récupère l'acide adique cristallisé avec un degréde pureté élevé (supérieur à 95 %).

Les autres diacides peuvent également être récupérés, après cristallisation de l'aicde adipique et concentration du mélange.

## Revendications

1. Procédé de traitement d'un matériau comprenant un polymère contenant au moins des fonctions amides, caractérisé en ce qu'il consiste à soumettre le matériau à une hydrolyse en présence de groupements nitreux dissouts dans le milieu d'hydrolyse et à une oxydation .

2. Procédé selon la revendication 1, caractérisé en ce que le milieu d'hydrolyse est un milieu oxydant.

3. Procédé selon la revendication 1 ou 2, caractérisé en ce que les groupements nitreux sont obtenus par décomposition thermique ou chimique d'au moins un composé comprenant des groupements NOₓ.

4. Procédé selon la revendication 3, caractérisé en ce que le composé précité est choisi dans le groupe comprenant l'acide nitrique, les nitrites d'alcalins ou métalliques, les vapeurs nitreuses .

5. Procédé selon l'une des revendications précédentes, caractérisé en ce que le milieu d'hydrolyse est une solution aqueuse à un pH acide, de préférence inférieur à 1 .

6. Procédé selon la revendication 5, caractérisé en ce que la concentration en H⁺ dans le milieu d'hydrolyse est au moins égale à 1 mole/l.

7. Procédé selon l'une des revendications précédentes, caractérisé en ce que la température d'hydrolyse est inférieure à 100°C, de préférence entre 40 et 100°C.

8. Procédé selon l'une des revendications précédentes, caractérisé en ce que l'hydrolyse est mise en oeuvre en présence d'un catalyseur d'oxydation.

9. Procédé selon la revendication 8, caractérisé en ce que le catalyseur est choisi dans le groupe comprenant les composés métalliques tels que des composés de cuivre, vanadium, titane, fer, cobalt, molybdène, nickel, ruthénium, manganèse, irridium .

10. Procédé selon l'une des revendications précédentes, caractérisé en ce que, après hydrolyse, les résidus solides sont éliminés du milieu d'hydrolyse et les acides dicarboxyliques produits sont extraits dudit milieu .

11. Procédé selon la revendication 10, caractérisé en ce que les acides dicarboxyliques produits sont extraits par cristallisation.

12. Procédé selon la revendication 10 ou 11, caractérisé en ce que les acides dicarboxyliques sont les acides dicarboxyliques comprenant un nombre de carbone égal à celui des monomères utilisés pour la synthèse du polymère et des acides dicarboxyliques comprenant un nombre inférieur de carbone.

13. Procédé selon l'une des revendications précédentes, caractérisé en ce que le polymère est choisi dans le groupe de polymères issus d'au moins un monomère comprenant des fonctions acides et/ou amines non liées directement à un noyau aromatique.

14. Procédé selon l'une des revendications précédentes, caractérisé en ce que le polymère est choisi dans le groupe comprenant les polyamides, polyétheramides, polyesteramides, polyétherestéramides, polyaramides les copolymères de ceux-ci et leurs mélanges.

15. Procédé selon la revendication 13 ou 14, caractérisé en ce que les polyamides sont choisis dans le groupe comprenant les polyamides obtenus par polycondensation d'une diamine avec un diacide, les polyamides obtenus par polycondensation d'un aminoacide, les copolyamides de ceux-ci et leurs mélanges.

16. Procédé selon la revendication 15, caractérisé en ce que les polyamides sont choisis dans le groupe comprenant le PA 6.6, PA 6.4, PA 6.11, PA 6.12, PA 4.6, PA 6, PA 12, PA 11.

17. Procédé selon l'une des revendications précédentes, caractérisé en ce que le matériau est constitué par un polymère ou un mélange de polymères.

18. Procédé selon l'une des revendications 1 à 17, caractérisé en ce que le matériau comprend un polymère, des additifs et des charges minérales ou organiques .

19. Procédé selon l'une des revendications précédentes, caractérisé en ce que le matériau est traité tel quel .

20. Procédé selon l'une quelconques des revendications 1 à 19, caractérisé en ce que le matériau est traité préalablement pour séparation avec d'autre matériau ou réduction en petites particules .

## Claims

1. Process for treating a material comprising a polymer containing at least amide functional groups, characterized in that it consists in subjecting the material to a hydrolysis in the presence of nitrous groups dissolved in the hydrolysis medium and to an oxidation.

2. Process according to Claim 1,characterized in that the hydrolysis medium is an oxidizing medium.

3. Process according to Claim 1 or 2, characterized in that the nitrous groups are obtained by thermal or chemical decomposition of at least one compound containing NOₓ groups.

4. Process according to Claim 3, characterized in that the abovementioned compound is chosen from the group including nitric acid, alkali metal or metal nitrites and nitrous vapours.

5. Process according to one of the preceding claims, characterized in that the hydrolysis medium is an aqueous solution with an acidic pH preferably lower than 1.

6. Process according to Claim 5, characterized in that the H⁺ concentration in the hydrolysis medium is at least 1 mole/l.

7. Process according to one of the preceding claims, characterized in that the temperature of hydrolysis is lower than 100°C, preferably between 40 and 100°C.

8. Process according to one of the preceding claims, characterized in that the hydrolysis is effected in the presence of an oxidation catalyst.

9. Process according to Claim 8, characterized in that the catalyst is chosen from the group including metal compounds such as copper, vanadium, titanium, iron, cobalt, molybdenum, nickel, ruthenium, manganese or iridium compounds.

10. Process according to one of the preceding claims, characterized in that, after hydrolysis, the solid residues are removed from the hydrolysis mixture and the dicarboxylic acids produced are extracted from the said mixture.

11. Process according to Claim 10, characterized in that the dicarboxylic acids produced are extracted by crystallization.

12. Process according to Claim 10 or 11, characterized in that the dicarboxylic acids are dicarboxylic acids containing a number of carbons equal to that of the monomers employed for the synthesis of the polymer and dicarboxylic acids containing a lower number of carbons.

13. Process according to one of the preceding claims, characterized in that the polymer is chosen from the group of polymers originating from at least one monomer containing acidic and/or amine functional groups not bonded directly to an aromatic nucleus.

14. Process according to one of the preceding claims, characterized in that the polymer is chosen from the group including polyamides, polyether amides, polyester amides, polyether ester amides, polyaramids, copolymers thereof and their mixtures.

15. Process according to Claim 13 or 14, characterized in that the polyamides are chosen from the group including the polyamides obtained by polycondensation of a diamine with a diacid, polyamides obtained by polycondensation of an amino acid, the copolyamides thereof and their mixtures.

16. Process according to Claim 15, characterized in that the polyamides are chosen from the group including PA 6.6, PA 6.4, PA 6.11, PA 6.12, PA 4.6, PA 6, PA 12 and PA 11.

17. Process according to one of the preceding claims, characterized in that the material consists of a polymer or a mixture of polymers.

18. Process according to one of Claims 1 to 17, characterized in that the material includes a polymer, additives and inorganic or organic fillers.

19. Process according to one of the preceding claims, characterized in that the material is treated as it is.

20. Process according to any one of Claims, 1 to 19, characterized in that the material is pretreated for separation from another material or reduction to small particles.

## Patentansprüche

1. Verfahren zur Behandlung eines Materials, das ein Polymer mit mindestens Amidfunktionen umfaßt, dadurch gekennzeichnet, daß es darin besteht, das Material einer Hydrolyse in Anwesenheit von in dem Hydrolysemedium gelösten Nitrosogruppen und einer Oxidation zu unterziehen.

2. Verfahren nach Anspruch 1, dadurch gekennzeichnet, daß das Hydrolysemedium ein oxidierendes Medium ist.

3. Verfahren nach Anspruch 1 oder 2, dadurch gekennzeichnet, daß die Nitrosogruppen durch thermische oder chemische Zersetzung von mindestens einer Verbindung erhalten werden, die Gruppen NOₓ umfaßt.

4. Verfahren nach Anspruch 3, dadurch gekennzeichnet, daß die vorstehend genannte Verbindung aus der Salpetersäure, die Alkalinitrite oder metallische Nitrite und nitrose Dämpfe umfassenden Gruppe gewählt wird.

5. Verfahren nach einem der vorstehenden Ansprüche, dadurch gekennzeichnet, daß das Hydrolysemedium eine wäßrige Lösung mit saurem pH-Wert ist, vorzugsweise von unter 1.

6. Verfahren nach Anspruch 5, dadurch gekennzeichnet, daß die Konzentration an H⁺ in dem Hydrolysemedium mindestens gleich 1 Mol/l beträgt.

7. Verfahren nach einem der vorstehenden Ansprüche, dadurch gekennzeichnet, daß die Temperatur der Hydrolyse unterhalb von 100 °C liegt, vorzugsweise zwischen 40 °C und 100 °C.

8. Verfahren nach einem der vorstehenden Ansprüche, dadurch gekennzeichnet, daß die Hydrolyse in Anwesenheit eines Oxidationskatalysators durchgeführt wird.

9. Verfahren nach Anspruch 8, dadurch gekennzeichnet, daß der Katalysator aus der die Metallverbindungen, wie die Verbindungen von Kupfer, Vanadium, Titan, Eisen, Kobalt, Molybdän, Nickel, Ruthenium, Mangan, Iridium, umfassenden Gruppe gewählt wird.

10. Verfahren nach einem der vorstehenden Ansprüche, dadurch gekennzeichnet, daß nach der Hydrolyse die festen Rückstände aus dem Hydrolysemedium entfernt und die entstandenen Dicarbonsäuren aus dem genannten Medium extrahiert werden.

11. Verfahren nach Anspruch 10, dadurch gekennzeichnet, daß die entstandenen Dicarbonsäuren durch Kristallisation extrahiert werden.

12. Verfahren nach Anspruch 10 oder 11, dadurch gekennzeichnet, daß die Dicarbonsäuren solche Dicarbonsäuren mit einer Anzahl von Kohlenstoffen sind, die gleich der ist, wie sie die bei der Synthese des Polymers verwendeten Monomeren und Dicarbonsäuren aufweisen, die ihrerseits eine niedrigere Anzahl von Kohlenstoffen umfassen.

13. Verfahren nach einem der vorstehenden Ansprüche, dadurch gekennzeichnet, daß das Polymer aus der Gruppe von Polymeren gewählt wird, die von mindestens einem Monomer abstammen, das Säurefunktionen und/oder Aminfunktionen umfaßt, die nicht direkt an einen aromatischen Kern gebunden sind.

14. Verfahren nach einem der vorstehenden Ansprüche, dadurch gekennzeichnet, daß das Polymer aus der die Polyamide, die Polyetheramide, die Polyesteramide, die Polyetheresteramide, die Polyaramide, deren Copolymere und ihre Mischungen umfassenden Gruppe gewählt wird.

15. Verfahren nach Anspruch 13 oder 14, dadurch gekennzeichnet, daß die Polyamide aus der Gruppe gewählt werden, die die durch Polykondensation eines Diamins mit einer Disäure erhaltenen Polyamide, die durch Polykondensation einer Aminosäure erhaltenen Polyamide, deren Copolyamide und ihre Mischungen umfaßt.

16. Verfahren nach Anspruch 15, dadurch gekennzeichnet, daß die Polyamide aus der PA 6.6, PA 6.4, PA 6.11, PA 6.12, PA 4.6, PA 6, PA 12, PA 11 umfassenden Gruppe gewählt werden.

17. Verfahren nach einem der vorstehenden Ansprüche, dadurch gekennzeichnet, daß das Material aus einem Polymer oder einer Mischung von Polymeren besteht.

18. Verfahren nach einem der Ansprüche 1 bis 17, dadurch gekennzeichnet, daß das Material ein Polymer, Zusatzstoffe und mineralische oder organische Füllstoffe umfaßt.

19. Verfahren nach einem der vorstehenden Ansprüche, dadurch gekennzeichnet, daß das Material so wie es ist behandelt wird.

20. Verfahren nach irgendeinem der Ansprüche 1 bis 19, dadurch gekennzeichnet, daß das Material zuvor mittels Trennung von anderem Material oder Überführung in kleine Teilchen behandelt wird.
